# EUROPEAN PATENT APPLICATION

(11) **EP 3 156 816 A1**
(43) Date of publication of application: **19.04.2017**
(21) Application number: 16161886.3
(22) Date of filing: 23.03.2016
(51) Int. Cl.: G01S 7/52, G01S 15/89, G10K 11/34

(54) **ULTRASOUND DEVICE AND METHOD OF PROCESSING ULTRASOUND SIGNAL**

(30) Priority: 12.10.2015 KR 20150142163
(71) Applicant: Samsung Medison Co., Ltd., Hongcheon-gun, Gangwon-do, 25108 (KR)
(72) Inventor: MIN, Kyou-sik, Gyeonggi-do (KR)
(74) Representative: Land, Addick Adrianus Gosling

(57) **Abstract**

An ultrasound device includes: a plurality of transducers constituting a two-dimensional probe; a signal processor configured to generate non-inverted signals based on first signals received from first transducers of a first group from among the plurality of transducers and to generate inverted signals based on second signals received from second transducers of a second group from among the plurality of transducers; a beamformer configured to generate a first summing signal (441) corresponding to the non-inverted signals by performing beamforming on the non-inverted signals and to generate a second summing signal (442) corresponding to the inverted signals by performing beamforming on the inverted signals; and a differential amplifier configured to differentially amplify the first summing signal (441) and the second summing signal (442). Switching noise (541, 551) which may be included in the first summing signal (441) and in the second summing signal (442) is removed in the differentially amplified signal (450).

## Description

This application claims the benefit of Korean Patent Application No. 10-2015-0142163, filed on October 12, 2015, in the Korean Intellectual Property Office, the disclosure of which is incorporated herein in its entirety by reference.

One or more exemplary embodiments relate to an ultrasound device and a method of processing an ultrasound signal, and more particularly, to an ultrasound device and a method of processing an ultrasound signal which may perform beamforming.

Ultrasound diagnosis apparatuses transmit ultrasound signals generated by transducers of a probe to an object and receive echo signals reflected from the object, thereby obtaining at least one image of an internal part of the object (e.g., soft tissue or blood flow). In particular, ultrasound diagnosis apparatuses are used for medical purposes including observation of the interior of an object, detection of foreign substances, and diagnosis of damage to the object. Such ultrasound diagnosis apparatuses provide high stability, display images in real time, and are safe due to the lack of radioactive exposure, compared to X-ray apparatuses. Therefore, ultrasound diagnosis apparatuses are widely used together with other image diagnosis apparatuses including a computed tomography (CT) apparatus, a magnetic resonance imaging (MRI) apparatus, and the like.

Additional aspects will be set forth in part in the description which follows and, in part, will be apparent from the description, or may be learned by practice of the presented embodiments.

According to one or more exemplary embodiments, an ultrasound device includes: a plurality of transducers constituting a two-dimensional (2D) probe; a signal processor configured to generate non-inverted signals based on first signals received from first transducers of a first group among the plurality of transducers and to generate inverted signals based on second signals received from second transducers of a second group among the plurality of transducers; a beamformer configured to generate a first summing signal corresponding to the non-inverted signals by performing beamforming on the non-inverted signals and to generate a second summing signal corresponding to the inverted signals by performing beamforming on the inverted signals; and a differential amplifier configured to differentially amplify the first summing signal and the second summing signal.

The first transducers of the first group may be different from the second transducers of the second group.

The signal processor may include a first low noise amplifier (LNA) configured to generate the non-inverted signals and a second LNA configured to generate the inverted signals.

The signal processor further includes a time-gain compensation (TGC) module configured to adjust a gain of at least one of the non-inverted signals and the inverted signals.

The beamformer may include a first delaying unit configured to adjust phases of the non-inverted signals and a second delaying unit configured to adjust phases of the inverted signals, wherein the beamformer generates the first summing signal by summing non-inverted signals whose phases are adjusted by the first delaying unit and generates the second summing signal by summing inverted signals whose phases are adjusted by the second delaying unit.

The first delaying unit may adjust the phases of the non-inverted signals by delaying at least one of the non-inverted signals by a predetermined period of time, and the second delaying unit may adjust the phases of the inverted signals by delaying at least one of the inverted signals by the predetermined period of time.

The first delaying unit may include a plurality of first capacitors respectively corresponding to the first transducers of the first group, and the second delaying unit may include a plurality of second capacitors respectively corresponding to the second transducers of the second group.

The first delaying unit may obtain a plurality of non-inverted signal values by sampling the non-inverted signals, store the plurality of non-inverted signal values in the plurality of first capacitors, and generate the non-inverted signals whose phases are adjusted by obtaining the plurality of non-inverted signal values from the plurality of first capacitors, and the second delaying unit may obtain a plurality of inverted signal values by sampling the inverted signals, store the plurality of inverted signal values in the plurality of second capacitors, and generate the inverted signals whose phases are adjusted by obtaining the plurality of inverted signal values from the plurality of second capacitors.

The 2D probe may include a plurality of sub-arrays, wherein the plurality of transducers constitute one sub-array among the plurality of sub-arrays included in the 2D probe.

The ultrasound device may further include an image processor configured to obtain differentially amplified signals respectively corresponding to the plurality of sub-arrays and to generate an ultrasound image based on the differentially amplified signals.

The ultrasound device may further include a controller configured to determine the first transducers of the first group and the second transducers of the second group in the plurality of transducers.

According to one or more exemplary embodiments, a method of processing a signal includes: generating non-inverted signals based on first ultrasound signals received from first transducers of a first group among a plurality of transducers constituting a two-dimensional (2D) probe; generating inverted signals based on second ultrasound signals received from second transducers of a second group among the plurality of transducers; generating a first summing signal corresponding to the non-inverted signals by performing beamforming on the non-inverted signals and generating a second summing signal corresponding to the inverted signals by performing beamforming on the inverted signals; and differentially amplifying the first summing signal and the second summing signal.

The generating of the non-inverted signals and the inverted signals may include: generating the non-inverted signals by using a first low noise amplifier (LNA); and generating the inverted signals by using a second LNA.

The generating of the non-inverted signals and the inverted signals may further include adjusting a gain of at least one of the non-inverted signals and the inverted signals.

The generating of the first summing signal may include: adjusting phases of the non-inverted signals; and generating the first summing signal by summing non-inverted signals whose phases are adjusted, and the generating of the second summing signal may include: adjusting phases of the inverted signals; and generating the second summing signal by summing inverted signals whose phases are adjusted.

The adjusting of the phases of the non-inverted signals may include adjusting the phases of the non-inverted signals by delaying at least one of the non-inverted signals by a predetermined period of time, and the adjusting of the phases of the inverted signals may include adjusting the phases of the inverted signals by delaying at least one of the inverted signals by a predetermined period of time.

The adjusting of the phases of the non-inverted signals may include: obtaining a plurality of non-inverted signal values by sampling the non-inverted signals; storing the plurality of non-inverted signal values in a plurality of first capacitors; and generating the non-inverted signals whose phases are adjusted by obtaining the plurality of non-inverted signal values from the plurality of first capacitors, and the adjusting of the phases of the inverted signals may include: obtaining a plurality of inverted signal values by sampling the inverted signals; storing the plurality of inverted signal values in a plurality of second capacitors; and generating the inverted signals whose phases are adjusted by obtaining the plurality of inverted signal values from the plurality of second capacitors.

The method may further include: obtaining differentially amplified signals respectively corresponding to a plurality of sub-arrays constituting the 2D probe; and generating an ultrasound image based on the differentially amplified signals.

The method may further include determining the first transducers of the first group and the second transducers of the second group in the plurality of transducers.

### BRIEF DESCRIPTION OF THE DRAWINGS

These and/or other aspects will become apparent and more readily appreciated from the following description of the embodiments, taken in conjunction with the accompanying drawings in which:
FIG. 1 is a view for explaining a two-dimensional (2D) probe and a sub-array, according to an exemplary embodiment;
FIG. 2 is a block diagram for explaining a first signal processing method according to an exemplary embodiment;
FIGS. 3A through 3C are diagrams for explaining a method of adjusting phases of non-inverted signals, according to the first signal processing method;
FIG. 4 is a block diagram for explaining a second signal processing method according to an exemplary embodiment;
FIGS. 5A through 5C are diagrams for explaining a method of adjusting phases of non-inverted signals and inverted signals, with respect to the second signal processing method;
FIG. 6 is a flowchart of a third signal processing method according to an exemplary embodiment;
FIG. 7 is a view for explaining first transducers of a first group and second transducers of a second group, according to an exemplary embodiment;
FIG. 8 is a block diagram for explaining the third signal processing method according to an exemplary embodiment;
FIGS. 9A and 9B are diagrams for explaining a method of adjusting phases of non-inverted signals and inverted signals, with respect to the third signal processing method;
FIG. 10 is a diagram for explaining the third signal processing method;
FIG. 11 is a view for explaining a process of obtaining an ultrasound image, according to an exemplary embodiment;
FIG. 12 is a table for comparing parameters of various signal processing methods, according to an exemplary embodiment;
FIG. 13 is a block diagram illustrating a configuration of an ultrasound device according to an exemplary embodiment;
FIG. 14 is a block diagram illustrating a configuration of the ultrasound device according to another exemplary embodiment; and
FIG. 15 is a block diagram illustrating a configuration of a wireless probe according to an exemplary embodiment.

### DETAILED DESCRIPTION

The terms used in this specification are those general terms currently widely used in the art in consideration of functions regarding the inventive concept, but the terms may vary according to the intention of one of ordinary skill in the art, precedents, or new technology in the art. Also, some terms may be arbitrarily selected by the applicant, and in this case, the meaning of the selected terms will be described in detail in the detailed description of the present specification. Thus, the terms used herein have to be defined based on the meaning of the terms together with the description throughout the specification.

Throughout the specification, it will also be understood that when a component "includes" an element, unless there is another opposite description thereto, it should be understood that the component does not exclude another element and may further include another element. In addition, terms such as "... unit", "... module", or the like refer to units that perform at least one function or operation, and the units may be implemented as hardware or software or as a combination of hardware and software.

Throughout the specification, an "ultrasound image" refers to an image of an object, which is obtained using ultrasound waves. Furthermore, an "object" may be a human, an animal, or a part of a human or animal. For example, the object may be an organ (e.g., the liver, the heart, the womb, the brain, a breast, or the abdomen), a blood vessel, or a combination thereof. Also, the object may be a phantom. The phantom means a material having a density, an effective atomic number, and a volume that are approximately the same as those of an organism. For example, the phantom may be a spherical phantom having properties similar to a human body.

Throughout the specification, a "user" may be, but is not limited to, a medical expert, for example, a medical doctor, a nurse, a medical laboratory technologist, or a medical imaging expert, or a technician who repairs medical apparatuses.

Hereinafter, exemplary embodiments will be described in detail with reference to the accompanying drawings.

As used herein, the term "and/or" includes any and all combinations of one or more of the associated listed items. Expressions such as "at least one of," when preceding a list of elements, modify the entire list of elements and do not modify the individual elements of the list.

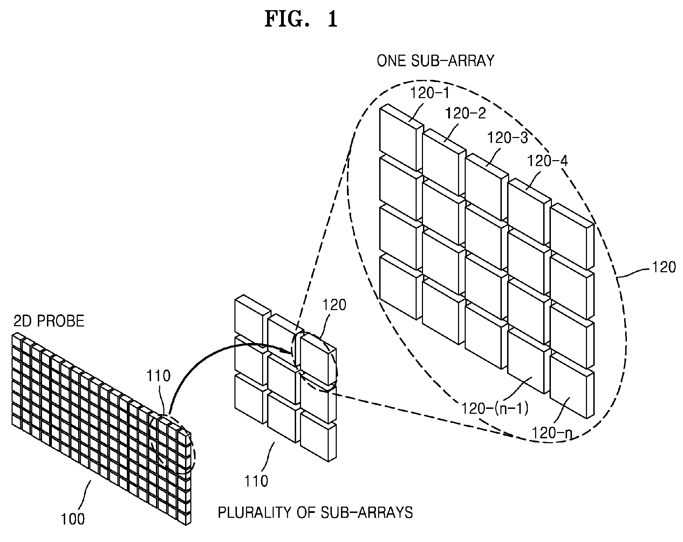

FIG. 1 is a view for explaining a two-dimensional (2D) probe and a sub-array according to an exemplary embodiment.

An ultrasound device may include a probe configured to emit an ultrasound signal to an object and to receive an echo signal reflected from the object. The probe may include a plurality of transducers. In this case, the probe may be one of a one-dimensional (1D) probe, a 2D probe, and a 3D probe. The following will be explained on the assumption that the ultrasound device includes a 2D probe 100.

Referring to FIG. 1, the 2D probe 100 may include a plurality of transducers that are, but are not limited to, 2D matrix transducers. Also, the number of transducers constituting the 2D probe 100 may vary according to the ultrasound device.

The number of transducers constituting the 2D probe 100 may be tens of times greater than that of a 1D probe. In this case, since the number of cables and related modules that are used to connect transducers of the 2D probe 100 and an inner system of the ultrasound device is also increased, it is difficult to perform beamforming. Accordingly, the ultrasound device may divide the 2D probe 100 into a plurality of sub-arrays 110. In this case, one sub-array 120 among the plurality of sub-arrays 110 may include a plurality of transducers, and the number of transducers constituting the sub-array 120 may vary according to the ultrasound device.

The plurality of sub-arrays 110 of FIG. 1 are some of sub-arrays constituting the 2D probe 100. Also, each of the sub-arrays constituting the 2D probe 100 may include a preset number of transducers. For example, as shown in FIG. 1, the sub-array 120 may include n transducers 120-1 through 120-n.

The ultrasound device may perform beamforming on each sub-array.

The term 'beamforming' refers to a process of increasing an intensity of an ultrasound signal by superposing ultrasound signals when transmitting and receiving the ultrasound signals by using a plurality of transducers. Since reflected ultrasound signals are attenuated and intensities of the reflected ultrasound signals are very low, it is difficult to generate an ultrasound image based on the reflected ultrasound signals. Accordingly, the ultrasound device may amplify an intensity of a signal by performing beamforming on reflected ultrasound signals. Hereinafter, for convenience of explanation, a signal output as a result of beamforming is referred to as a beamforming signal.

The ultrasound device including the 2D probe 100 may perform beamforming on each of the plurality of sub-arrays 110. Hereinafter, for convenience of explanation, beamforming performed on each of the plurality of sub-arrays 110 is referred to as micro-beamforming. Also, a signal output as a result of micro-beamforming is referred to as a micro-beamforming signal.

Times taken for ultrasound signals to be emitted from transducers to an object and then to be reflected from the object are different from one another. Accordingly, when ultrasound signals received from a plurality of transducers are superposed, since phases of the received signals are different from one another, an amplified signal may not be obtained. Accordingly, the ultrasound device needs to adjust phases of ultrasound signals and amplify signals whose phases are adjusted.

The ultrasound device may obtain a micro-beamforming signal by performing micro-beamforming on each of the plurality of sub-arrays 110 and may perform beamforming based on the micro-beamforming signals of the plurality of sub-arrays 110. Accordingly, the ultrasound device may reduce the number of cables and related modules used to connect transducers and an inner system of the ultrasound device.

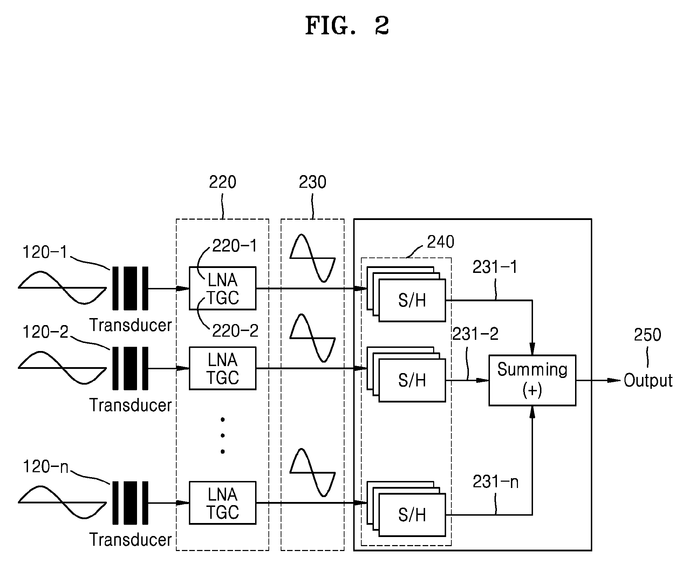

A process of performing beamforming on one sub-array will now be explained in detail with reference to FIGS. 2 through 3C.

FIG. 2 is a block diagram for explaining a first signal processing method according to an exemplary embodiment.

FIG. 2 will be explained on the assumption that the number of transducers constituting one sub-array 120 is n.

According to an exemplary embodiment, the ultrasound device may generate non-inverted signals 230 based on ultrasound echo signals received from the plurality of transducers 120-1 through 120-n. In order to generate the non-inverted signals 230, the ultrasound device may amplify the ultrasound echo signals. In this case, the ultrasound device may use, but is not limited to, low noise amplifiers (LNAs) 220-1 in order to amplify the ultrasound echo signals.

Also, the ultrasound device may adjust a gain of at least one of the ultrasound echo signals by using time-gain compensation (TGC) modules 220-2.

Ultrasound signals are attenuated while being propagated. In this case, an ultrasound echo signal reflected from tissue that is far from a surface of an object may be more attenuated than an ultrasound echo signal reflected from tissue that is close to the surface of the object and may take more time to reach the 2D probe 100. Accordingly, the ultrasound device may use the TGC modules 220-2 in order to compensate for an attenuation difference in time.

In this case, the LNAs 220-1 and the TGC modules 220-2 may be provided as one hardware module 220 or may be separately provided.

Also, the ultrasound device may adjust phases of the non-inverted signals 230 generated through the LNAs 220-1 and the TGC modules 220-2.

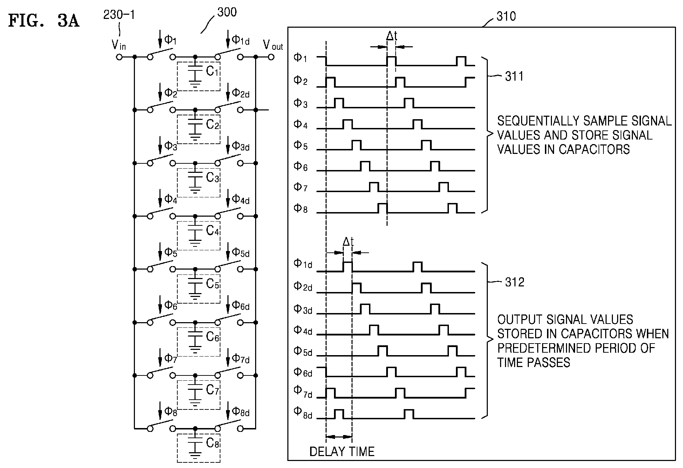

As shown in FIG. 2, the ultrasound device may use, but is not limited to, sample and hold circuits 240 in order to adjust phases of the non-inverted signals 230. A method of adjusting phases of the non-inverted signals 230 will be explained below in detail with reference to FIGS. 3A through 3C.

The ultrasound device may generate a summing signal 250 by summing non-inverted signals 231-1 through 231-n whose phases are adjusted. In this case, the summing signal 250 may be a beamforming signal of one sub-array 120.

FIG. 3A is a diagram for explaining a sample and hold circuit 300 according to the first signal processing method.

As shown in FIG. 3A, the sample and hold circuit 300 may include a plurality of switches and capacitors.

According to an exemplary embodiment, the ultrasound device may obtain a plurality of signal values by sampling the non-inverted signals 230 for every predetermined period of time and may store the plurality of signal values in a plurality of capacitors. In this case, the number of times sampling which is performed per unit time may vary according to the ultrasound device. FIGS. 3A through 3C will be explained on the assumption that sampling is performed 8 times per unit time.

The term 'sampling' may refer to a process of obtaining continuous signal values for every predetermined period of time. For example, the ultrasound device may obtain signal values at predetermined points of time for every period of time Δt according to a clock signal 311 of FIG. 3A. For convenience of explanation, the following will be explained on the assumption that the number of times a signal is sampled is a sampling number.

The sample and hold circuit 300 of FIG. 3A corresponds to one non-inverted signal 230-1.

Referring to FIG. 3A, the ultrasound device may sample the non-inverted signal 230-1 for every predetermined period of time and may store the non-inverted signal 230-1 in capacitors C₁ through C₈. In this case, the ultrasound device may sample the non-inverted signal 230-1 according to the clock signal 311 corresponding to Φ₁ through Φ₈ and may store the non-inverted signal 230-1 in the plurality of capacitors (e.g., C₁ through C₈).

For example, when Φ₁ is HIGH, a switch for connecting the non-inverted signal 230-1 and the capacitor C₁ may be closed and a first value of the sampled non-inverted signal 230-1 may be stored in the capacitor C₁. Also, after Δt, when Φ2 is HIGH, a switch for connecting the non-inverted signal 230-1 and the capacitor C₂ may be closed and a second value of the sampled non-inverted signal 230-1 may be stored in the capacitor C₂.

Also, the ultrasound device may obtain the stored values of the non-inverted signal 230-1 from the capacitors (e.g., C₁ through C₈) according to a clock signal 312 corresponding to Φ_{1d} through Φ_{8d}.

For example, when a predetermined delay time passes after a value of the non-inverted signal 230-1 that is sampled at a first point of time is stored in the capacitor C₁, Φ_{1d} is "HIGH". Then, a switch for connecting the capacitor C₁ and an output end of the sample and hold circuit 300 may be closed and the ultrasound device may obtain the first value of the non-inverted signal 230-1 stored in the capacitor C₁. Values of the non-inverted signal 230-1 obtained by the ultrasound device may be sampled values, and the ultrasound device may generate the non-inverted signal 231-1 whose phase is adjusted based on the obtained values of the non-inverted signal 230-1.

As shown in FIG. 3A, when a sampling number per unit time is 8, the ultrasound device may require 8 capacitors (e.g., C₁ through C₈) in order to obtain the non-inverted signal 231-1 whose phase is adjusted.

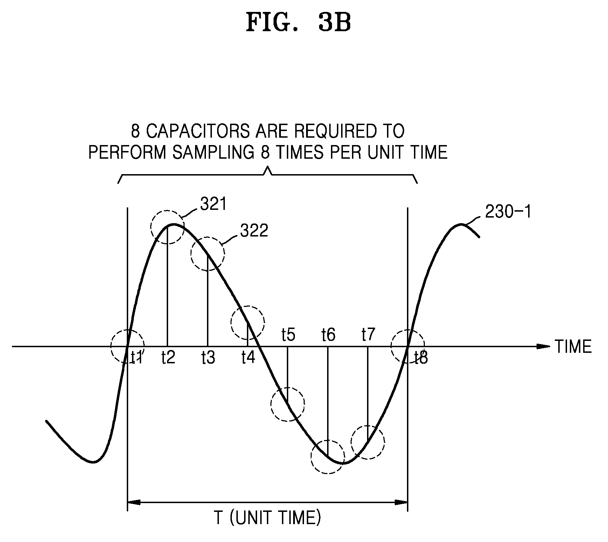

FIG. 3B is a graph for explaining a process of sampling a non-inverted signal according to an exemplary embodiment.

Referring to FIG. 3B, when a sampling number per unit time is 8, the ultrasound device may sample a value of the non-inverted signal 230-1 at points of time t1 through t8. For example, when sampling is performed at the point of time t2, as shown in an area 321, the second value of the non-inverted signal 230-1 corresponding to the point of time t2 may be sampled and the sampled second value may be stored in the capacitor C₂. After a predetermined delay time passes, the ultrasound device may obtain the stored second value from the capacitor C₂. In this case, since a switch is turned on/off according to a clock signal, a switching noise may be generated.

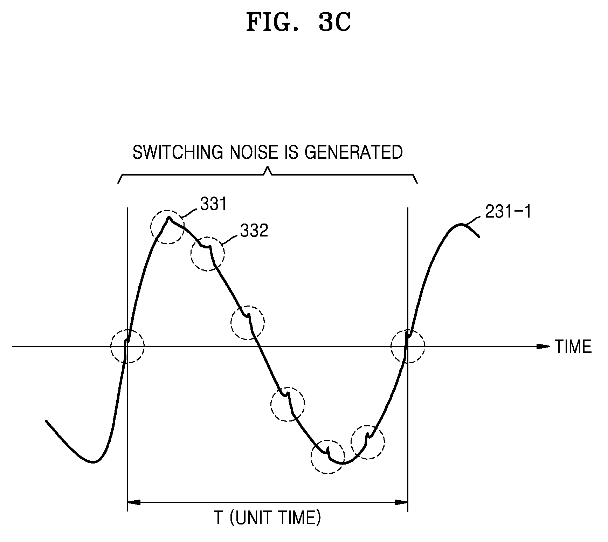

FIG. 3C is a graph for explaining a switching noise according to an exemplary embodiment.

A signal of FIG. 3C may be the non-inverted signal 231-1 whose phase is adjusted. As described above, when the ultrasound device obtains stored non-inverted signals from capacitors, since a switch is turned on/off according to a clock signal, the non-inverted signal 231-1 whose phase is adjusted may include a switching noise.

Referring to FIG. 3C, when the ultrasound device obtains stored signal values from capacitors (e.g., C₁ through C₈), switching may be performed 8 times and the non-inverted signal 231-1 whose phase is adjusted may include switching noises (e.g., 331 and 332). Accordingly, the summing signal 250 obtained by summing the non-inverted signals (e.g., 231-1 through 231-n) whose phases are adjusted in FIG. 2 may also include a switching noise.

The first signal processing method described with reference to FIGS. 2 through 3C has a problem in that a non-inverted signal whose phase is adjusted may include a switching noise as described above.

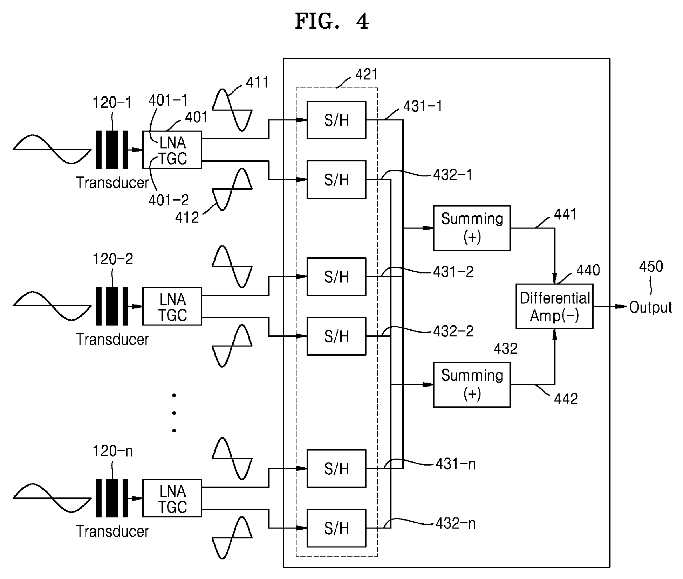

A second signal processing method for removing a switching noise will now be explained with reference to FIGS. 4 through 5C.

FIG. 4 is a block diagram for explaining the second signal processing method according to an exemplary embodiment.

A LNA 401-1, a TGC module 401-2, and a sample and hold circuit 421 of FIG. 4 may respectively correspond to the LNA 220-1, the TGC module 220-2, and the sample and hold circuit 240 of FIG. 2, and thus a repeated explanation thereof will not be given.

The ultrasound device may generate inverted signals and non-inverted signals based on signals received from the plurality of transducers 120-1 through 120-n by using a LNA and TGC module 401.

For example, referring to FIG. 4, the ultrasound device may generate a non-inverted signal 411 and an inverted signal 412 of a signal received from the transducer 120-1 by using the LNA and TGC module 401. There may be a phase difference of 180° between the inverted signal 412 and the non-inverted signal 411.

The ultrasound device may adjust phases of the non-inverted signal 411 and the inverted signal 412 by using the sample and hold circuits 421.

The ultrasound device may generate non-inverted signals (e.g., 431-1 through 431-n) whose phases are adjusted and inverted signals (e.g., 432-1 through 432-n) whose phases are adjusted for each of n transducers (e.g., 120-1 through 120-n) constituting one sub-array 120. Next, the ultrasound device may generate a non-inverted summing signal 441 by summing the non-inverted signals 431-1 through 431-n whose phases are adjusted and may generate an inverted summing signal 442 by summing the inverted signals 432-1 through 432-n whose phases are adjusted.

The ultrasound device may generate a beamforming signal 450 of one sub-array 120 by differentially amplifying the non-inverted summing signal 441 and the inverted summing signal 442.

When the non-inverted summing signal 441 and the inverted summing signal 442 are differentially amplified, it may mean, but is not limited to, that the inverted summing signal 442 is subtracted from the non-inverted summing signal 441 and a signal obtained by subtracting the inverted summing signal 442 from the non-inverted summing signal 441 is amplified.

As described above, each of the non-inverted summing signal 441 and the inverted summing signal 442 may include a switching noise. Also, since the non-inverted signals 431-1 through 431-n whose phases are adjusted and the inverted signals 432-1 through 432-n whose phases are adjusted are generated based on the same clock signal, a switching noise may be generated at the same point of time. Accordingly, the ultrasound device may remove a switching noise by differentially amplifying the non-inverted summing signal 441 and the inverted summing signal 442.

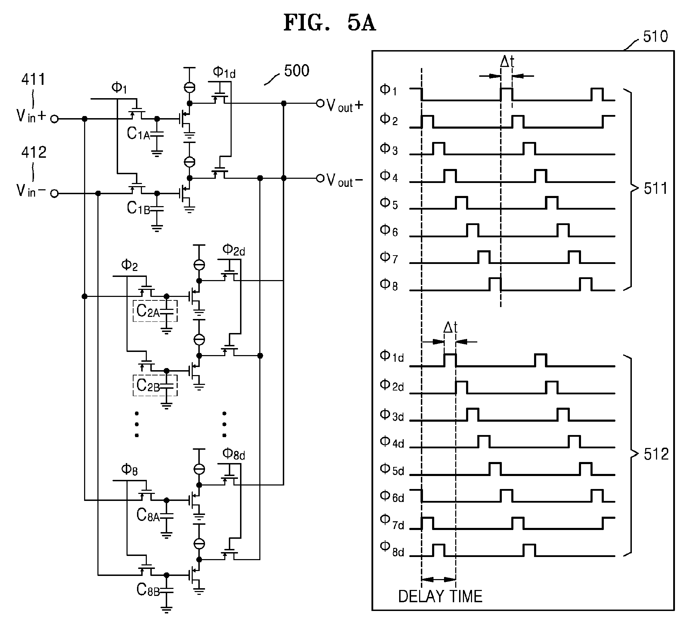

FIG. 5A is a diagram for explaining a sample and hold circuit 500 according to the second signal processing method.

The sample and hold circuit 500 of FIG. 5A corresponds to the non-inverted signal 411 and the inverted signal 412. In this case, the non-inverted signal 411 and the inverted signal 412 may be signals generated based on an ultrasound echo signal received from the transducer 120-1 of FIG. 4. FIG. 5A will be explained on the assumption that a sampling number per unit time is 8. The sample and hold circuit 500 has been described in detail with reference to FIG. 3A, and thus a repeated explanation thereof will not be given.

The ultrasound device may adjust phases of the non-inverted signal 411 and the inverted signal 412 according to the same clock signal 510 of FIG. 5A.

For example, referring to FIG. 5A, the ultrasound device may sample the non-inverted signal 411 and the inverted signal 412 based on a clock signal 511 corresponding to Φ₁ through Φ₈ for every predetermined period of time and may store values of the sampled non-inverted signal 411 and values of the sampled inverted signal 412 in capacitors (e.g., C_{1A} through C_{8A} and C_{1B} through C_{8B}). In this case, the values of the sampled non-inverted signal 411 may be stored in the capacitors C_{1A} through C_{8A} and the values of the sampled inverted signal 412 may be stored in the capacitors C_{1B} through C_{8B}.

Also, the ultrasound device may obtain the stored values of the non-inverted signal 411 and the inverted signal 412 from the capacitors C_{1A} through C_{8A} and C_{1B} through C_{8B} based on a clock signal 512 corresponding to Φ_{1d} through Φ_{8d}.

The ultrasound device may sample a non-inverted signal and an inverted signal according to the same clock signal and may store the non-inverted signal and the inverted signal in the capacitors C_{1A} through C_{8A} and C_{1B} through C_{8B}, and may obtain the stored signals from the capacitors C_{1A} through C_{8A} and C_{1B} through C_{8B}. Accordingly, a non-inverted signal whose phase is adjusted and an inverted signal whose phase is adjusted may include a switching noise at the same point of time.

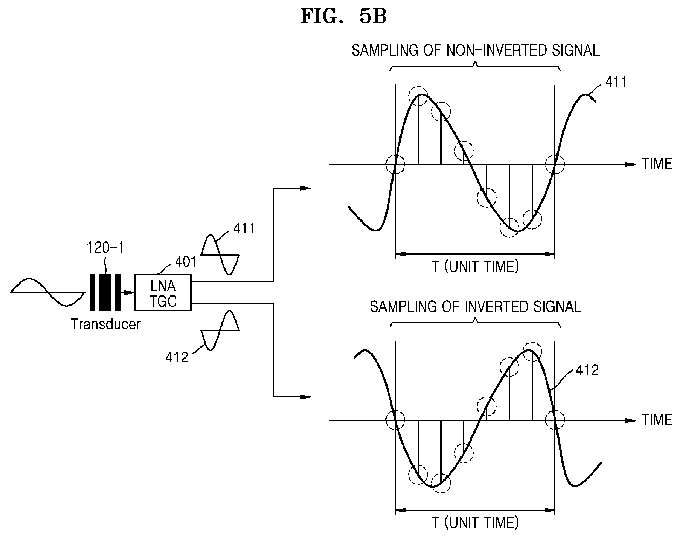

FIG. 5B is a diagram for explaining a process of sampling a non-inverted signal and an inverted signal according to an exemplary embodiment.

The ultrasound device may generate a non-inverted signal and an inverted signal for each of the transducers 120-1 through 120-n constituting one sub-array 120. For convenience of explanation, in FIG. 5B, the non-inverted signal 411 and the inverted signal 412 generated based on a signal received from one transducer 120-1 are sampled.

Referring to FIG. 5B, the ultrasound device may separately sample the non-inverted signal 411 and the inverted signal 412. In this case, although the non-inverted signal 411 and the inverted signal 412 are sampled by different sample and hold circuits 421, the non-inverted signal 411 and the inverted signal 412 are sampled according to the same clock signal.

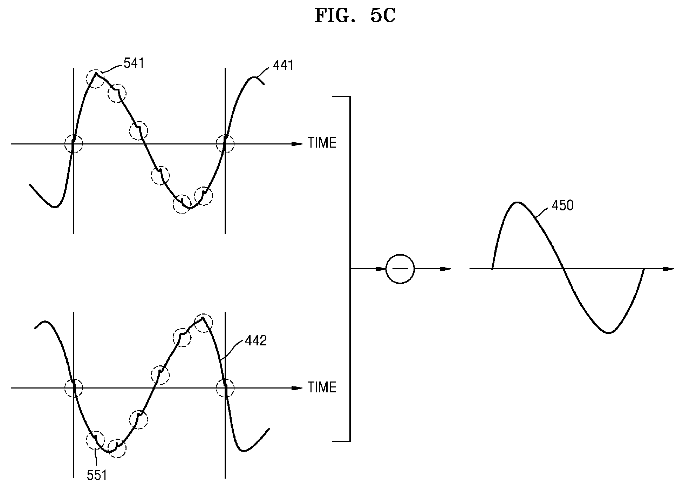

FIG. 5C is a graph for explaining a process of removing a switching noise according to the second signal processing method according to an exemplary embodiment.

According to an exemplary embodiment, a process performed by the ultrasound device to delay signals stored in capacitors by a predetermined period of time and then output the signals may include a process of turning on/off a switch. In this case, a switching noise may be generated when the switch is turned on/off. Accordingly, each of the non-inverted signals 431-1 through 431-n whose phases are adjusted and the inverted signals 432-2 through 432-n whose phases are adjusted may include a switching noise. Also, each of the non-inverted summing signal 441 and the inverted summing signal 442 respectively obtained by summing the non-inverted signals 431-1 through 431-n whose phases are adjusted and the inverted signals 432-1 through 432-n whose phases are adjusted may include a switching noise.

For example, referring to FIG. 5C, the non-inverted summing signal 441 and the inverted summing signal 442 may respectively include a first switching noise 541 and a second switching noise 551.

The ultrasound device may remove a switching noise by differentially amplifying the non-inverted summing signal 441 and the inverted summing signal 442. Since the non-inverted summing signal 441 and the inverted summing signal 442 may include a switching noise at the same point of time as described above, a switching noise that is commonly generated in the non-inverted summing signal 441 and the inverted summing signal 442 may be offset by differentially amplifying the non-inverted summing signal 441 and the inverted summing signal 442. For example, the first switching noise 541 corresponding to the non-inverted summing signal 441 and the second switching noise 551 corresponding to the inverted summing signal 442 may be offset.

Although a method of adjusting a phase of a signal by using a sample and hold circuit has been described with reference to FIGS. 5A through 5C, the present exemplary embodiment is not limited thereto.

When a sampling number per unit time is n, the second signal processing method of FIG. 4 requires a process of sampling a non-inverted signal and an inverted signal n times per unit time. Accordingly, the ultrasound device requires 2*n capacitors in order to obtain a non-inverted signal whose phase is adjusted and an inverted signal whose phase is adjusted.

When the second signal processing method of FIG. 4 and the first signal processing method of FIG. 2 are compared with each other, the second signal processing method may remove a switching noise whereas the first signal processing method may not remove a switching noise. Also, the second signal processing method requires capacitors the number of which is twice or more greater than that in the first signal processing method.

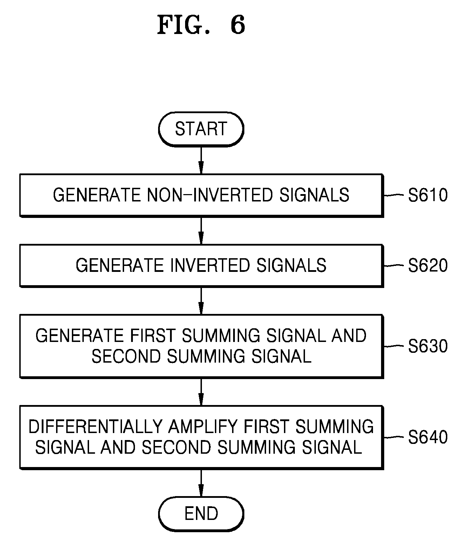

A third signal processing method of removing a switching noise by using capacitors the number of which is the same as a sampling number per unit time will now be explained with reference to FIGS. 6 through 11.

FIG. 6 is a flowchart of the third signal processing method according to an exemplary embodiment.

The flowchart of FIG. 6 shows a method of processing signals received from a plurality of transducers constituting one sub-array.

In operation S610, the ultrasound device may generate non-inverted signals based on first ultrasound signals received from first transducers of a first group among a plurality of transducers constituting a 2D probe.

In operation S620, the ultrasound device may generate inverted signals based on second ultrasound signals received from second transducers of a second group among the plurality of transducers.

The first transducers of the first group and the second transducers of the second group may constitute one sub-array among a plurality of sub-arrays included in the 2D probe.

Also, the first transducers of the first group and the second transducers of the second group in the one sub-array may be different from each other. For example, transducers in odd columns in the one sub-array may be designated as the first transducers of the first group and transducers in even columns in the sub-array may be designated as the second transducers of the second group.

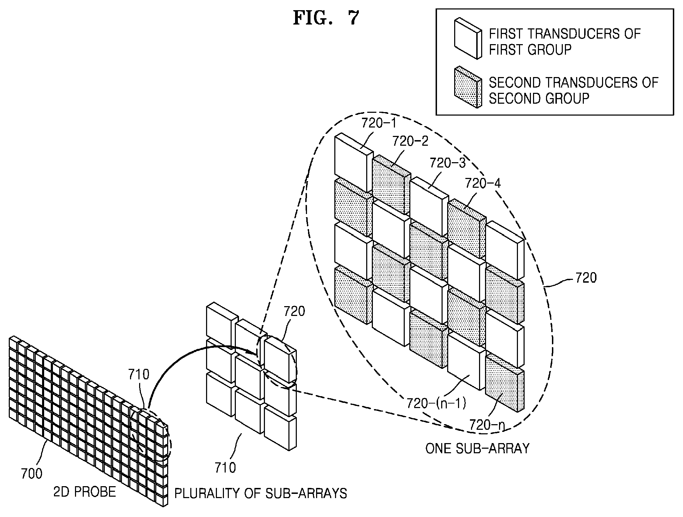

The first transducers of the first group and the second transducers of the second group will be explained below in detail with reference to FIG. 7.

Also, the ultrasound device may use a first LNA in order to generate the non-inverted signals based on the first ultrasound signals, and may use a second LNA in order to generate the inverted signals based on the second ultrasound signals. A gain of each of the first LNA and the second LNA may be equal to or greater than 1.

Also, the ultrasound device may adjust a gain of at least one of the non-inverted signals and the inverted signals by using a TGC module.

In operation S630, the ultrasound device may generate a first summing signal corresponding to the non-inverted signals and may generate a second summing signal corresponding to the inverted signals. For example, the ultrasound device may generate the first summing signal by performing beamforming on the non-inverted signals and may generate the second summing signal by performing beamforming on the inverted signals.

The term 'beamforming' may include an operation of adjusting phases of signals and an operation of summing the signals whose phases are adjusted. For example, the ultrasound device may adjust a phase by delaying at least one of a plurality of signals by a predetermined period of time. Also, the ultrasound device may use, but is not limited to, a sample and hold circuit in order to generate delayed signals. The sample and hold circuit may correspond to the sample and hold circuit 240 of FIG. 2.

In operation S640, the ultrasound device may differentially amplify the first summing signal and the second summing signal.

Each of the first summing signal and the second summing signal generated in operation S630 may include at least one switching noise due to switching that is performed for a predetermined period of time. Accordingly, the ultrasound device may remove a switching noise by differentially amplifying the first summing signal and the second summing signal.

Also, the third signal processing method generates a non-inverted signal or an inverted signal for each transducer. Accordingly, the third signal processing method requires only capacitors the number of which is the same as a sampling number per unit time in order to adjust a phase of the non-inverted signal or the inverted signal.

FIG. 7 is a view for explaining first transducers of a first group and second transducers of a second group according to an exemplary embodiment.

Referring to FIG. 7, the ultrasound device may divide a plurality of transducers 720-1 through 720-n constituting one sub-array 720 into first transducers 720-1, 720-3, ..., and 720-(n-1) of a first group and second transducers 720-2, 720-4, ..., and 720-n of a second group.

For example, the ultrasound device may divide the transducers 720-1 through 720-n into, but not limited thereto, the first transducers 720-1, 720-3, ..., and 720-(n-1) of the first group and the second transducers 720-2, 720-4, ..., and 720-n of the second group by dividing the transducers 720-1 through 720-n into odd and even transducers according to an order in which the transducers 720-1 through 720-n are arranged.

According to an exemplary embodiment, the number of the first transducers 720-1, 720-3, ..., and 720-(n-1) of the first group and the number of the second transducers 720-2, 720-4, ..., and 720-n of the second group may be the same or different from each other.

Also, according to an exemplary embodiment, the ultrasound device may re-designate the first transducers 720-1, 720-3, ..., and 720-(n-1) of the first group and the number of the second transducers 720-2, 720-4, ..., and 720-n of the second group in the sub-array 720.

The second signal processing method of FIG. 4 may generate a non-inverted signal and an inverted signal for each of transducers constituting one sub-array. However, the third signal processing method of FIG. 7 may divide transducers constituting one sub-array into two groups and may generate a non-inverted signal or an inverted signal according to a group in which each transducer is included.

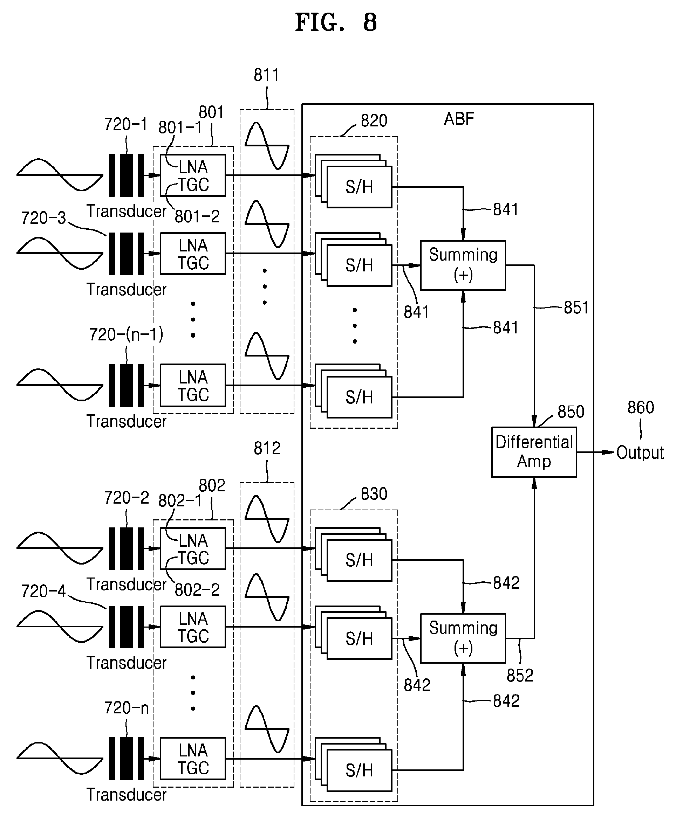

FIG. 8 is a block diagram for explaining the third signal processing method according to an exemplary embodiment. A LNA and TGC module 801 and a sample and hold circuit 820 have been described above in detail, and thus a repeated explanation thereof will not be given.

Referring to FIG. 8, the ultrasound device may generate non-inverted signals 811 by using a first LNA 801-1 corresponding to each of the first transducers 720-1, 720-3, ..., and 720-(n-1) of the first group. Also, the ultrasound device may adjust a gain of at least one of the generated non-inverted signals 811. The ultrasound device may use, but is not limited to, a TGC module 801-2 in order to adjust a gain.

The ultrasound device may generate inverted signals 812 by using a second LNA 802-1 corresponding to each of the second transducers 720-2, 720-4, ..., and 720-n of the second group, and may adjust a gain of at least one of the inverted signals 812.

The ultrasound device may separately perform beamforming on the non-inverted signals 811 and the inverted signals 812. For example, the ultrasound device may adjust phases of the non-inverted signals 811 and may generate a first summing signal 851 by summing non-inverted signals 841 whose phases are adjusted. Likewise, the ultrasound device may adjust phases of the inverted signals 812 and may generate a second summing signal 852 by summing inverted signals 842 whose phases are adjusted.

Next, the ultrasound device may obtain a beamforming signal 860 of one sub-array 720 by differentially amplifying the first summing signal 851 and the second summing signal 852 by using a differential amplifier 850.

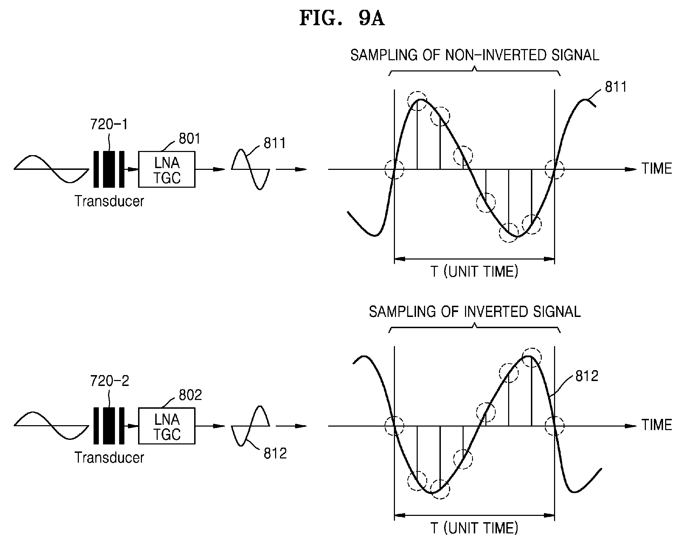

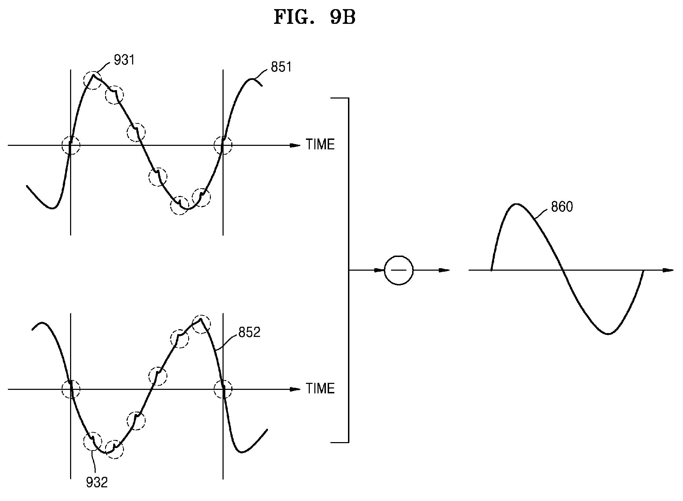

An operation of storing a non-inverted signal and an inverted signal in capacitors or obtaining the non-inverted signal and the inverted signal from the capacitors in order to adjust a phase will now be explained with reference to FIGS. 9A and 9B.

FIG. 9A is a graph for explaining a sampling process according to an exemplary embodiment.

Referring to FIG. 9A, the ultrasound device may sample the non-inverted signal 811 corresponding to a first ultrasound signal received from the first transducer 720-1 included in the first group and the inverted signal 812 corresponding to a second ultrasound signal received from the second transducer 720-2 included in the second group. In this case, since the first ultrasound signal is an echo signal received through the first transducer 720-1 and the second ultrasound signal is an echo signal received from the second transducer 720-2, the first ultrasound signal and the second ultrasound signal may be different from each other.

According to an exemplary embodiment, when a sampling number per unit time is 8, although the non-inverted signal 811 and the inverted signal 812 are sampled respectively by the sample and hold circuits 820 and 830, the inverted signal 811 and the inverted signal 812 may be sampled according to the same clock signal.

For example, the ultrasound device may sample the non-inverted signal 811 and may store the non-inverted signal 811 in first capacitors, and may sample the inverted signal 812 and may store the inverted signal 812 in second capacitors. Also, the ultrasound device may obtain the non-inverted signal 811 and the inverted signal 812 stored in the first capacitors and the second capacitors according to the same clock signal.

The third signal processing method may require capacitors the number of which is half the number in the second signal processing method.

For example, according to the second signal processing method, the ultrasound device generates a first non-inverted signal and a first inverted signal for a first ultrasound signal and generates a second non-inverted signal and a second inverted signal for a second ultrasound signal. Since the ultrasound device samples and stores the first non-inverted signal, the first inverted signal, the second non-inverted signal, and the second inverted signal (when a sampling number per unit time is 8), the second signal processing method requires 32 capacitors.

However, according to the third signal processing method, the ultrasound device generates a first non-inverted signal corresponding to a first ultrasound signal and generates a second inverted signal corresponding to a second ultrasound signal, and then samples and stores the first non-inverted signal and the second inverted signal (when a sampling number per unit time is 8), the ultrasound device requires 16 capacitors.

FIG. 9B is a graph for explaining a process of removing a switching noise according to the third signal processing method.

As described above, according to the third signal processing method, the first summing signal 851 and the second summing signal 852 may respectively include a first switching noise 931 and a second switching noise 932 as shown in FIG. 9B. Since a switch is turned on/or off based on the same clock signal, the first summing signal 851 and the second summing signal 852 may include a switching noise at the same point of time. Accordingly, the ultrasound device may remove a switching noise by differentially amplifying the first summing signal 851 and the second summing signal 852.

The beamforming signal 860 obtained by differentially amplifying the first summing signal 851 and the second summing signal 852 may be a beamforming signal of one sub-array 720.

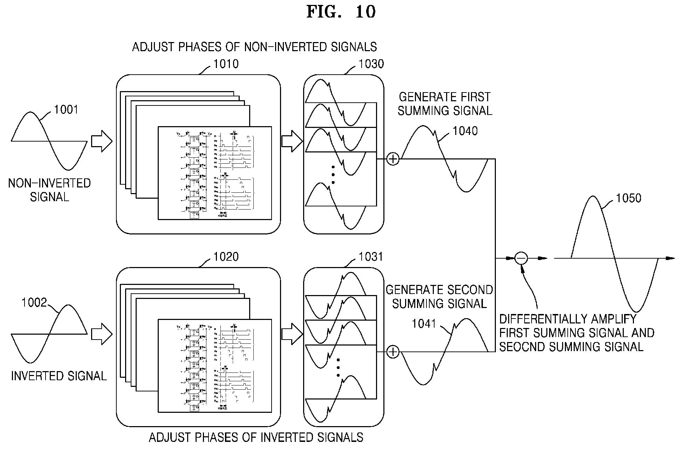

FIG. 10 is a diagram for explaining the third signal processing method according to an exemplary embodiment.

FIG. 10 shows a process of processing a signal of FIG. 8 by using an ultrasound signal.

The ultrasound device may generate non-inverted signals 1001 based on first signals received from the first transducers 720-1, 720-3, ..., and 720-(n-1) of the first group and may generate inverted signals 1002 based on second signals received from the second transducers 720-2, 720-4, ..., and 720-n of the second group.

The ultrasound device may generate non-inverted signals 1030 whose phases are adjusted by using sample and hold circuits 1010 respectively corresponding to the non-inverted signals 1001. Also, the ultrasound device may generate inverted signals 1031 whose phases are adjusted by using sample and hold circuits 1020 respectively corresponding to the inverted signals 1002.

The ultrasound device may generate a first summing signal 1040 by summing the non-inverted signals 1030 whose phases are adjusted and may generate a second summing signal 1041 by summing the inverted signals 1031 whose phases are adjusted. In this case, each of the first summing signal 1040 and the second summing signal 1041 may include a switching noise as described above.

The ultrasound device may obtain a beamforming signal 1050 of one sub-array 720 by differentially amplifying the first summing signal 1040 and the second summing signal 1041.

Accordingly, the ultrasound device may reduce the number of required capacitors by generating a non-inverted signal or an inverted signal according to a group in which each transducer is included. Also, the ultrasound device may also remove a switching noise by differentially amplifying the first summing signal 1040 and the second summing signal 1041.

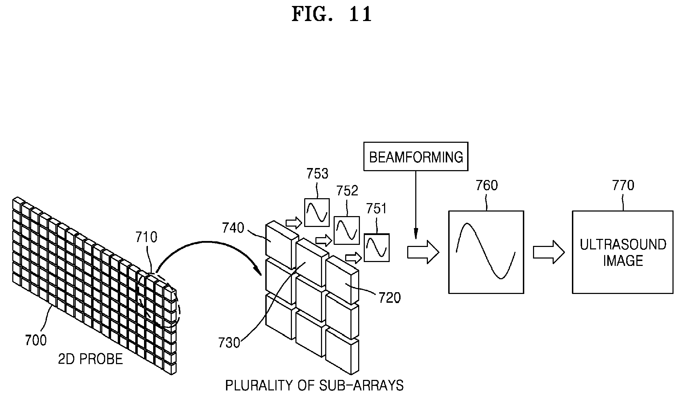

Although a process of obtaining a beamforming signal of one sub-array has been described with reference to FIGS. 2 through 10, an operation performed by the ultrasound device to obtain an ultrasound image by using beamforming signals corresponding to a plurality of sub-arrays will now be explained with reference to FIG. 11.

FIG. 11 is a graph for explaining a process of obtaining an ultrasound image according to an exemplary embodiment.

Referring to FIG. 11, the ultrasound device including a 2D probe 700 may include a plurality of sub-arrays 710. Accordingly, the ultrasound device may obtain beamforming signals respectively corresponding to the plurality of sub-arrays 710 by performing micro-beamforming on each of the plurality of sub-arrays 710. The following will focus on three sub-arrays 720, 730, and 740 among the plurality of sub-arrays 710 constituting the 2D probe 700, for convenience of explanation.

The ultrasound device may generate beamforming signals 751, 752, and 753 respectively corresponding to the sub-arrays 720 730, and 740.

The ultrasound device may obtain a final beamforming signal 760 by performing beamforming on the beamforming signals 751, 752, and 753 respectively corresponding to the sub-arrays 720, 730, and 740. Accordingly, the ultrasound device may obtain an ultrasound image 770 based on the final beamforming signal 760.

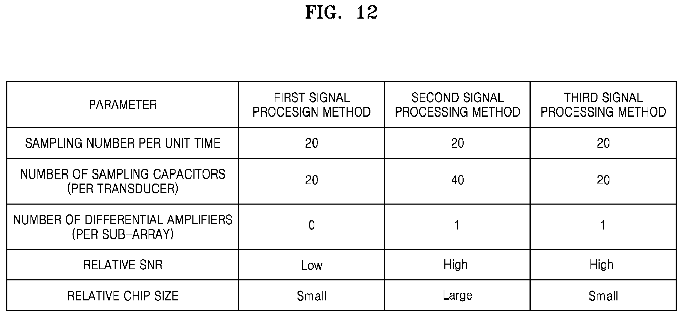

FIG. 12 is a table for comparing parameters of various signal processing methods of FIGS. 2 through 11. For convenience of explanation, the following will be explained on the assumption that a sampling number per unit time is 20 in each signal processing method.

In order to generate a non-inverted signal and an inverted signal based on a signal received from one transducer, the first signal processing method of FIG. 2 and the third signal processing method of FIG. 8 require 20 capacitors. However, since the second signal processing method of FIG. 4 generates a non-inverted signal and an inverted signal for one transducer, the second signal processing method requires 40 capacitors.

While the first signal processing method does not include differential amplification, the second signal processing method and the third signal processing method differentially amplify two summing signals and thus each require one differential amplifier.

Since the second signal processing method and the third signal processing method remove a switching noise as described above, unlike the first signal processing method, the second signal processing method and the third signal processing method have a relatively high signal-to-noise ratio (SNR).

Also, since the first signal processing method and the third signal processing method require fewer capacitors than the second signal processing method, the first signal processing method and the third signal processing method may have a relatively small chip size.

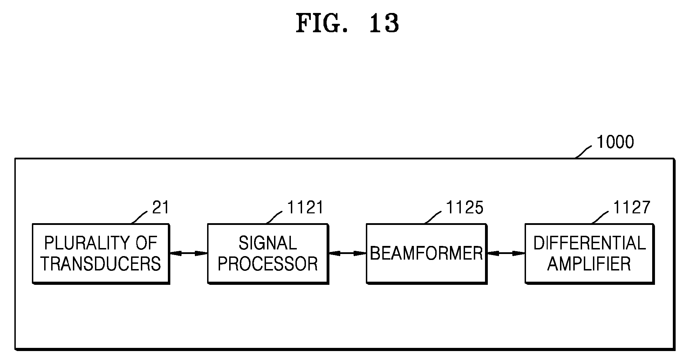

FIG. 13 is a block diagram illustrating a configuration of an ultrasound device 1000 according to an exemplary embodiment.

The ultrasound device 1000 may include a plurality of transducers 21, a signal processor 1121, a beamformer 1125, and a differential amplifier 1127. However, all of the elements are not essential elements. The ultrasound device 100 may include less or more elements than the elements shown in FIG. 13.

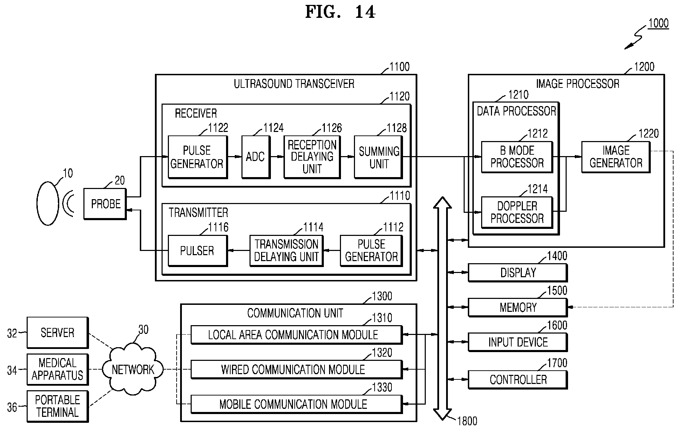

For example, as shown in FIG. 14, the ultrasound device 1000 according to an exemplary embodiment may include a probe 20, an ultrasound transceiver 1100, an image processor 1200, a communication unit 1300, a display 1400, a memory 1500, an input device 1600, and a controller 1700, which may be connected to one another via buses 1800.

The ultrasound device 1000 may be a cart type device or a portable type device. Examples of the portable ultrasound device may include, but are not limited to, a picture archiving and communication system (PACS) viewer, a smartphone, a laptop computer, a personal digital assistant (PDA), and a tablet PC.

The probe 20 transmits ultrasound waves to an object 10 in response to a driving signal applied by the ultrasound transceiver 1100 and receives echo signals reflected by the object 10. The probe 20 includes the plurality of transducers 21, and the plurality of transducers 21 oscillate in response to electric signals and generate acoustic energy, that is, ultrasound waves. Furthermore, the probe 20 may be connected to the main body of the ultrasound device 1000 by wire or wirelessly, and according to embodiments, the ultrasound device 1000 may include a plurality of the probes 20.

Also, the probe 20 may include the 2D probe 100 or 700 according to the ultrasound device 1000. In this case, the ultrasound device 1000 may include the plurality of transducers 21 constituting the 2D probe 100 or 700.

A transmitter 1110 supplies a driving signal to the probe 20. The transmitter 110 includes a pulse generator 1112, a transmission delaying unit 1114, and a pulser 1116. The pulse generator 1112 generates pulses for forming transmission ultrasound waves based on a predetermined pulse repetition frequency (PRF), and the transmission delaying unit 1114 delays the pulses by delay times necessary for determining transmission directionality. The pulses which have been delayed correspond to a plurality of piezoelectric vibrators included in the probe 20, respectively. The pulser 1116 applies a driving signal (or a driving pulse) to the probe 20 based on timing corresponding to each of the pulses which have been delayed.

A receiver 1120 generates ultrasound data by processing echo signals received from the probe 20. The receiver 120 may include an amplifier 1122, an analog-to-digital converter (ADC) 1124, a reception delaying unit 1126, and a summing unit 1128. The amplifier 1122 amplifies echo signals in each channel, and the ADC 1124 performs analog-to-digital conversion on the amplified echo signals.

The amplifier 1122 may include the signal processor 1121 of FIG. 13. Accordingly, the amplifier 1122 may include a LNA and TGC module and may generate a non-inverted signal or an inverted signal.

The reception delaying unit 1126 delays digital echo signals output by the ADC 1124 by delay times necessary for determining reception directionality, and the summing unit 1128 generates ultrasound data by summing the echo signals processed by the reception delaying unit 1126. In some embodiments, the receiver 1120 may not include the amplifier 1122. In other words, if the sensitivity of the probe 20 or the capability of the ADC 1124 to process bits is enhanced, the amplifier 1122 may be omitted.

The reception delaying unit 1126 may include the beamformer 1125 of FIG. 13. According to an exemplary embodiment, when the probe 20 is the 2D probe 100 or 700, the beamformer 1125 may include a micro-beamformer corresponding to each sub-array.

The beamformer 1125 may include a first delaying unit configured to adjust phases of non-inverted signals and a second delaying unit configured to adjust phases of inverted signals.

Also, the beamformer 1125 may generate a first summing signal by summing non-inverted signals whose phases are adjusted by the first delaying unit and may generate a second summing signal by summing inverted signals whose phases are adjusted by the second delaying unit. Each of the first delaying unit and the second delaying unit may include, but is not limited to, a sample and hold circuit in order to obtain signals whose phases are adjusted. Also, the first delaying unit may include first capacitors respectively corresponding to first transducers of a first group and the second delaying unit may include second capacitors respectively corresponding to second transducers of a second group.

Also, the summing unit 1128 may include the differential amplifier 1127 of FIG. 13. The ultrasound device 1000 may differentially amplify the first summing signal and the second summing signal by using the differential amplifier 1127.

The image processor 1200 generates an ultrasound image by scan-converting ultrasound data generated by the ultrasound transceiver 1100. The ultrasound image may be not only a grayscale ultrasound image obtained by scanning an object in an amplitude (A) mode, a brightness (B) mode, and a motion (M) mode, but also a Doppler image showing a movement of an object via a Doppler effect. The Doppler image may be a blood flow Doppler image showing flow of blood (also referred to as a color Doppler image), a tissue Doppler image showing a movement of tissue, or a spectral Doppler image showing a moving speed of an object as a waveform.

Also, when the ultrasound device 1000 includes a 2D probe, the image processor 1200 may generate an ultrasound image based on a beamforming signal corresponding to each of a plurality of sub-arrays.

A B mode processor 1212 extracts B mode components from ultrasound data and processes the B mode components. An image generator 1220 may generate an ultrasound image indicating signal intensities as brightness based on the extracted B mode components 1212.

Similarly, a Doppler processor 1214 may extract Doppler components from ultrasound data, and the image generator 1220 may generate a Doppler image indicating a movement of an object as colors or waveforms based on the extracted Doppler components.

According to an exemplary embodiment, the image generator 1220 may generate a three-dimensional (3D) ultrasound image via volume-rendering with respect to volume data and may also generate an elasticity image by imaging deformation of the object 10 due to pressure. Furthermore, the image generator 1220 may display various pieces of additional information in an ultrasound image by using text and graphics. In addition, the generated ultrasound image may be stored in the memory 1500.

The display 1400 displays the generated ultrasound image. The display 1400 may display not only an ultrasound image, but also various pieces of information processed by the ultrasound device 1000 on a screen image via a graphical user interface (GUI). In addition, the ultrasound device 1000 may include two or more displays 1400 according to embodiments.

The communication unit 1300 is connected to a network 30 by wire or wirelessly to communicate with an external device or a server. The communication unit 1300 may exchange data with a hospital server or another medical apparatus in a hospital, which is connected thereto via a PACS. Furthermore, the communication unit 1300 may perform data communication according to the digital imaging and communications in medicine (DICOM) standard.

The communication unit 1300 may transmit or receive data related to diagnosis of an object, e.g., an ultrasound image, ultrasound data, and Doppler data of the object, via the network 30 and may also transmit or receive medical images captured by another medical apparatus, e.g., a computed tomography (CT) apparatus, a magnetic resonance imaging (MRI) apparatus, or an X-ray apparatus. Furthermore, the communication unit 1300 may receive information about a diagnosis history or medical treatment schedule of a patient from a server and utilizes the received information to diagnose the patient. Furthermore, the communication unit 1300 may perform data communication not only with a server or a medical apparatus in a hospital, but also with a portable terminal of a medical doctor or patient.

The communication unit 1300 is connected to the network 30 by wire or wirelessly to exchange data with a server 32, a medical apparatus 34, or a portable terminal 36. The communication unit 1300 may include one or more components for communication with external devices. For example, the communication unit 1300 may include a local area communication module 1310, a wired communication module 1320, and a mobile communication module 1330.

The local area communication module 1310 refers to a module for local area communication within a predetermined distance. Examples of local area communication techniques according to an exemplary embodiment may include, but are not limited to, wireless LAN, Wi-Fi, Bluetooth, ZigBee, Wi-Fi Direct (WFD), ultra wideband (UWB), infrared data association (IrDA), Bluetooth low energy (BLE), and near field communication (NFC).

The wired communication module 1320 refers to a module for communication using electric signals or optical signals. Examples of wired communication techniques according to an exemplary embodiment may include communication via a twisted pair cable, a coaxial cable, an optical fiber cable, and an Ethernet cable.

The mobile communication module 1330 transmits or receives wireless signals to or from at least one selected from a base station, an external terminal, and a server on a mobile communication network. The wireless signals may be voice call signals, video call signals, or various types of data for transmission and reception of text/multimedia messages.

The memory 1500 stores various data processed by the ultrasound device 1000. For example, the memory 1500 may store medical data related to diagnosis of an object, such as ultrasound data and an ultrasound image that are input or output, and may also store algorithms or programs which are to be executed in the ultrasound device 1000.

The memory 1500 may be any of various storage media, e.g., a flash memory, a hard disk drive, EEPROM, etc. Furthermore, the ultrasound device 1000 may utilize web storage or a cloud server that performs the storage function of the memory 1500 online.

The input device 1600 refers to a means via which a user inputs data for controlling the ultrasound device 1000. The input device 1600 may include hardware components, such as a keypad, a mouse, a touch pad, a touch screen, and a jog switch. However, exemplary embodiments are not limited thereto, and the input device 1600 may further include any of various other input units including an electrocardiogram (ECG) measuring module, a respiration measuring module, a voice recognition sensor, a gesture recognition sensor, a fingerprint recognition sensor, an iris recognition sensor, a depth sensor, a distance sensor, etc.

The controller 1700 may control all operations of the ultrasound device 1000. In other words, the controller 1700 may control operations among the probe 20, the ultrasound transceiver 1100, the image processor 1200, the communication unit 1300, the display 1400, the memory 1500, and the input device 1600 shown in FIG. 14.

The controller 1700 may determine the first transducers of the first group and the second transducers of the second group in the plurality of transducers 21.

For example, the controller 1700 may designate transducers in odd columns in one sub-array as the first transducers of the first group and may designate transducers in even columns in the sub-array as the second transducers of the second group. Alternatively, the controller 1700 may designate transducers in odd rows as the first transducers of the first group and may designate transducers in even rows as the second transducers of the second group. Alternatively, the controller 1700 may designate transducers so that the first transducers of the first group and the second transducers of the second group intersect each other. However, the present exemplary embodiment is not limited thereto.

Also, the controller 1700 may re-designate the first transducers of the first group and the second transducers of the second group in one sub-array.

All or some of the probe 20, the ultrasound transceiver 1100, the image processor 1200, the communication unit 1300, the display 1400, the memory 1500, the input device 1600, and the controller 1700 may be implemented as software modules. Also, at least one of the ultrasound transceiver 1100, the image processor 1200, and the communication unit 1300 may be included in the control unit 1600; however, the present exemplary embodiment is not limited thereto.

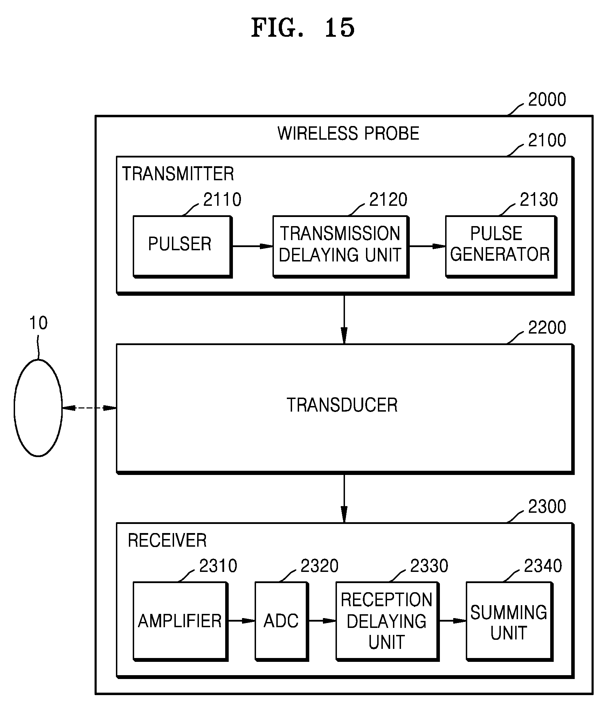

FIG. 15 is a block diagram illustrating a configuration of a wireless probe 2000 according to an exemplary embodiment. As described above with reference to FIG. 14, the wireless probe 2000 may include a plurality of transducers, and, according to embodiments, may include some or all of the components of the ultrasound transceiver 1100 shown in FIG. 14.

The wireless probe 2000 according to the embodiment shown in FIG. 15 includes a transmitter 2100, a transducer 2200, and a receiver 2300. Since descriptions thereof are given above with reference to FIG. 14, detailed descriptions thereof will be omitted here. In addition, according to exemplary embodiments, the wireless probe 2000 may selectively include a reception delaying unit 2330 and a summing unit 2340.

The wireless probe 2000 may transmit ultrasound signals to the object 10, receive echo signals from the object 10, generate ultrasound data, and wirelessly transmit the ultrasound data to the ultrasound device 1000 shown in FIG. 1.

The signal processing method of the inventive concept may be implemented as computer instructions which may be executed by various computer means, and recorded on a computer-readable recording medium. The computer-readable recording medium may include program commands, data files, data structures, or a combination thereof. The program commands recorded on the computer-readable recording medium may be specially designed and constructed for the inventive concept or may be known to and usable by one of ordinary skill in a field of computer software. Examples of the computer-readable medium include storage media such as magnetic media (e.g., hard discs, floppy discs, or magnetic tapes), optical media (e.g., compact disc-read only memories (CD-ROMs), or digital versatile discs (DVDs)), magneto-optical media (e.g., floptical discs), and hardware devices that are specially configured to store and carry out program commands (e.g., ROMs, RAMs, or flash memories). Examples of the program commands include a high-level language code that may be executed by a computer using an interpreter as well as a machine language code made by a compiler.

While one or more embodiments have been described with reference to the figures, it will be understood by one of ordinary skill in the art that various changes in form and details may be made therein without departing from the spirit and scope of the inventive concept as defined by the following claims.

## Claims

1. An ultrasound device comprising:
a plurality of transducers constituting a two-dimensional (2D) probe;
a signal processor configured to generate non-inverted signals based on first signals received from first transducers of a first group from among the plurality of transducers and to generate inverted signals based on second signals received from second transducers of a second group from among the plurality of transducers;
a beamformer configured to generate a first summing signal corresponding to the non-inverted signals by performing beamforming on the non-inverted signals and to generate a second summing signal corresponding to the inverted signals by performing beamforming on the inverted signals; and
a differential amplifier configured to differentially amplify the first summing signal and the second summing signal.

2. The ultrasound device of claim 1, wherein the first transducers of the first group are different from the second transducers of the second group.

3. The ultrasound device of claim 1 or 2, wherein the signal processor comprises a first low noise amplifier (LNA) configured to generate the non-inverted signals and a second LNA configured to generate the inverted signals.

4. The ultrasound device of claim 3, wherein the signal processor further comprises a time-gain compensation (TGC) module configured to adjust a gain of at least one of the non-inverted signals and the inverted signals.

5. The ultrasound device of any of claims 1-4, wherein the beamformer comprises a first delaying unit configured to adjust phases of the non-inverted signals and a second delaying unit configured to adjust phases of the inverted signals,
wherein the beamformer generates the first summing signal by summing non-inverted signals whose phases are adjusted by the first delaying unit and generates the second summing signal by summing inverted signals whose phases are adjusted by the second delaying unit.

6. The ultrasound device of claim 5, wherein the first delaying unit adjusts the phases of the non-inverted signals by delaying at least one of the non-inverted signals by a predetermined period of time, and
the second delaying unit adjusts the phases of the inverted signals by delaying at least one of the inverted signals by the predetermined period of time.

7. The ultrasound device of claim 5 or 6, wherein the first delaying unit comprises a plurality of first capacitors respectively corresponding to the first transducers of the first group, and
the second delaying unit comprises a plurality of second capacitors respectively corresponding to the second transducers of the second group.

8. The ultrasound device of claim 7, wherein the first delaying unit obtains a plurality of non-inverted signal values by sampling the non-inverted signals, stores the plurality of non-inverted signal values in the plurality of first capacitors, and generates the non-inverted signals whose phases are adjusted by obtaining the plurality of non-inverted signal values from the plurality of first capacitors, and
the second delaying unit obtains a plurality of inverted signal values by sampling the inverted signals, stores the plurality of inverted signal values in the plurality of second capacitors, and generates the inverted signals whose phases are adjusted by obtaining the plurality of inverted signal values from the plurality of second capacitors.

9. The ultrasound device of any of claims 1-8, wherein the 2D probe comprises a plurality of sub-arrays,
wherein the plurality of transducers constitute one sub-array among the plurality of sub-arrays included in the 2D probe.

10. The ultrasound device of claim 9, further comprising an image processor configured to obtain differentially amplified signals respectively corresponding to the plurality of sub-arrays and to generate an ultrasound image based on the differentially amplified signals.

11. The ultrasound device of any of claims 1-10, further comprising a controller configured to determine the first transducers of the first group and the second transducers of the second group from among the plurality of transducers.

12. A method of processing a signal, the method comprising:
generating non-inverted signals based on first ultrasound signals received from first transducers of a first group from among a plurality of transducers constituting a two-dimensional (2D) probe;
generating inverted signals based on second ultrasound signals received from second transducers of a second group from among the plurality of transducers;
generating a first summing signal corresponding to the non-inverted signals by performing beamforming on the non-inverted signals and generating a second summing signal corresponding to the inverted signals by performing beamforming on the inverted signals; and
differentially amplifying the first summing signal and the second summing signal.

13. The method of claim 12, wherein the generating of the non-inverted signals and the inverted signals comprises:
generating the non-inverted signals by using a first low noise amplifier (LNA); and
generating the inverted signals by using a second LNA.

14. The method of claim 12 or 13, wherein the generating of the first summing signal comprises:
adjusting phases of the non-inverted signals; and
generating the first summing signal by summing non-inverted signals whose phases are adjusted, and
the generating of the second summing signal comprises:
adjusting phases of the inverted signals; and
generating the second summing signal by summing inverted signals whose phases are adjusted.

15. The method of claim 14, wherein the adjusting of the phases of the non-inverted signals comprises adjusting the phases of the non-inverted signals by delaying at least one of the non-inverted signals by a predetermined period of time, and
the adjusting of the phases of the inverted signals comprises adjusting the phases of the inverted signals by delaying at least one of the inverted signals by a predetermined period of time.
